# EUROPÄISCHE PATENTSCHRIFT

(11) **EP 4 750 624 B1**
(45) Veröffentlichungstag und Bekanntmachung des Hinweises auf die Patenterteilung: **28.10.2026**
(21) Anmeldenummer: 24748876.0
(22) Anmeldetag: 25.07.2024
(51) Int. Cl.: B29D 11/00, B29C 64/124, B29D 11/02, B33Y 10/00, B33Y 30/00, B33Y 50/00, B33Y 80/00

(54) **VERFAHREN UND ANORDNUNG ZUM HERSTELLEN EINER INTRAOKULARLINSE**
METHOD AND ASSEMBLY FOR PRODUCING AN INTRAOCULAR LENS
PROCÉDÉ ET ENSEMBLE DE PRODUCTION DE LENTILLE INTRAOCULAIRE

(30) Priorität: 26.07.2023 DE 102023207114
(43) Veröffentlichungstag der Anmeldung: 03.06.2026
(73) Patentinhaber: Carl Zeiss Meditec AG, 07745 Jena (DE)
(72) Erfinder: THALLER, Michael, 73447 Oberkochen (DE); SCHREIBER, Benjamin, 73447 Oberkochen (DE); BADUR, Thorben, 73447 Oberkochen (DE); NICOLI, Francesca, 73447 Oberkochen (DE); MASCH, Jennifer-magdalena, 73447 Oberkochen (DE)
(74) Vertreter: Patentanwälte Bressel und Partner mbB
(86) Internationale Anmeldenummer: PCT/EP2024/071100
(87) Internationale Veröffentlichungsnummer: WO 2025/021910

(56) Entgegenhaltungen:
- DE-B3- 102020 108 375
- US-A1- 2014 264 981
- US-A1- 2018 001 581

## Beschreibung

Die Erfindung betrifft ein Verfahren und eine Anordnung zum Herstellen einer Intraokularlinse.

Intraokularlinsen werden herkömmlich durch Drehen hergestellt. Hierzu wird zuerst durch Polymerisation das Material der Intraokularlinse hergestellt. Anschließend werden aus dem Material Rohlinge geschnitten. Bei hydrophilen Intraokularlinsen werden die Rohlinge insbesondere mittels eines Wachses an einer Drehmaschine befestigt, bei hydrophoben Intraokularlinsen werden die Rohlinge insbesondere bei etwa -20°C angefroren. Ein computergesteuerter Roboterarm, der mit einer Diamantspitze bestückt ist, spant aus dem in der Drehmaschine rotierenden Rohling die Intraokularlinse. Hierbei handelt es sich jedoch um ein aufwändiges und kostenintensives Verfahren. Zudem ist von Nachteil, dass eine beim Drehen verwendete Diamantspitze Rillen auf der Oberfläche der Intraokularlinse hinterlässt, wobei die Rillen eine optische Qualität der Intraokularlinse beeinträchtigen.

Aus der DE 10 2020 108 375 B3 ist ferner ein Verfahren zum Herstellen einer Intraokularlinse im Wege des tomographischen Druckens bekannt. Das Verfahren umfasst die Schritte: Bereitstellen eines für elektromagnetische Strahlung transparenten Behälters, in dem eine Flüssigkeit angeordnet ist, die mit der elektromagnetischen Strahlung aushärtbar ist; Bestrahlen der Flüssigkeit mit einem Satz an Bildern, die von der elektromagnetischen Strahlung gebildet werden und die jeweils eine Intraokularlinse zeigen, wobei jedes der Bilder des Satzes unter einem anderen Einstrahlwinkel bezüglich einer durch die Flüssigkeit verlaufenden Bezugsebene in die Flüssigkeit eingestrahlt wird, wodurch die Flüssigkeit ausgehärtet wird und die ausgehärtete Flüssigkeit die Intraokularlinse bildet, wobei in der Flüssigkeit ein Aktuator, ein Solarmodul und/oder ein Sensor angeordnet ist und die Intraokularlinse um den Aktuator, das Solarmodul und/oder den Sensor herum gebildet wird.

Es ist ferner bekannt, einen Hohlraum in Intraokularlinsen mit einer Flüssigkeit zu befüllen. Dies erlaubt das Verändern und Einstellen der optischen Eigenschaften der Intraokularlinse.

Der Erfindung liegt die Aufgabe zu Grunde, ein Verfahren und eine Anordnung zum Herstellen einer Intraokularlinse zu verbessern.

Die Aufgabe wird erfindungsgemäß durch ein Verfahren mit den Merkmalen des Patentanspruchs 1 und eine Anordnung mit den Merkmalen des Patentanspruchs 10 gelöst. Vorteilhafte Ausgestaltungen der Erfindung ergeben sich aus den Unteransprüchen.

Es ist einer der Grundgedanken der Erfindung, einen Hohlraum in der Intraokularlinse mit derjenigen Flüssigkeit zu befüllen, die beim tomographischen Drucken verwendet wird, nach dem tomographischen Drucken aber deren Fähigkeit zum Aushärten zu deaktivieren. Hierdurch kann die in dem Hohlraum verbleibende Flüssigkeit insbesondere nicht mehr durch elektromagnetische Strahlung ausgehärtet werden. Insbesondere kann eine weitere ungewollte Polymerisation hierdurch verhindert werden, sodass die Flüssigkeit im Anschluss im flüssigen Zustand verbleibt. Insbesondere wird hierbei eine aktive Gruppe des Monomers (z.B. Acrylat) in eine inerte Gruppe umgewandelt. Dem liegt der Gedanke zugrunde, dass durch das Verwenden der Technik des tomographischen Druckens die Intraokularlinse in einer (mechanisch) stabilisierenden Lösung gedruckt werden kann, welche durch die aushärtbare Flüssigkeit bereitgestellt wird. Wird beim tomographischen Drucken (mindestens) ein Hohlraum in der Intraokularlinse hergestellt, so verbleibt die aushärtbare Flüssigkeit nach dem Beenden des Druckvorgangs in dem (mindestens) einen Hohlraum, da eine Aktivierung der aushärtbaren Flüssigkeit zur Polymerisation dort nicht stattgefunden hat, die aushärtbare Flüssigkeit den Hohlraum aber auch nicht mehr verlassen kann, da die Flüssigkeit von den Wänden des (mindestens einen) Hohlraums eingeschlossen wird. Um ein nachfolgendes ungewolltes Aushärten bzw. eine weitere Polymerisation zu verhindern, wird die in dem (mindestens einen) Hohlraum verbleibende aushärtbare Flüssigkeit deaktiviert und der flüssige Zustand wird beibehalten.

Insbesondere wird ein Verfahren zum Herstellen einer Intraokularlinse zur Verfügung gestellt, umfassend: Bereitstellen eines für elektromagnetische Strahlung transparenten Behälters, Bereitstellen einer mittels elektromagnetischer Strahlung aushärtbaren Flüssigkeit in dem Behälter, Erzeugen und/oder Bereitstellen eines Datensatzes aus Bildern einer Intraokularlinse mit mindestens einem Hohlraum, wobei die Bilder Projektionen der Intraokularlinse mit dem mindestens einen Hohlraum aus unterschiedlichen Richtungen beinhalten, tomographisches Drucken der aushärtbaren Flüssigkeit mittels elektromagnetischer Strahlung ausgehend von dem erzeugten und/oder bereitgestellten Datensatz zum Ausbilden der Intraokularlinse mit dem mindestens einen Hohlraum, und Deaktivieren der Fähigkeit zum Aushärten der aushärtbaren Flüssigkeit in dem mindestens einen Hohlraum.

Ferner wird insbesondere eine Anordnung zum Herstellen einer Intraokularlinse geschaffen, umfassend einen für elektromagnetische Strahlung transparenten Behälter; eine Datenverarbeitungseinrichtung, die eingerichtet ist zum Erzeugen und/oder Bereitstellen eines Datensatzes aus Bildern einer Intraokularlinse mit mindestens einem Hohlraum, wobei die Bilder Projektionen der Intraokularlinse mit dem mindestens einen Hohlraum aus unterschiedlichen Richtungen beinhalten; eine tomographische Druckeinrichtung, die eingerichtet ist zum tomographischen Drucken einer in dem Behälter bereitgestellten aushärtbaren Flüssigkeit mittels elektromagnetischer Strahlung ausgehend von dem erzeugten und/oder bereitgestellten Datensatz zum Ausbilden der Intraokularlinse mit dem mindestens einen Hohlraum; und mindestens ein Deaktivierungsmittel, das eingerichtet ist zum Deaktivieren der Fähigkeit zum Aushärten der aushärtbaren Flüssigkeit in dem mindestens einen Hohlraum oder das Deaktivieren vorzubereiten und/oder zu unterstützen.

Ein Vorteil des Verfahrens und der Anordnung ist, dass ein Hohlraum in der Intraokularlinse nicht mehr gesondert mit einer Flüssigkeit befüllt werden muss. Hierdurch kann das Problem einer Leckage, welches bei einer nachträglichen Befüllung stets besteht, vermieden werden, da ein Zugang zum Hohlraum und ein anschließender Wiederverschluss nicht mehr notwendig sind.

Die elektromagnetische Strahlung liegt insbesondere im optischen Wellenlängenbereich, insbesondere im sichtbaren und/oder UV-Wellenlängenbereich. Die aushärtbare Flüssigkeit kann insbesondere die Eigenschaften aufweisen, welche in der DE 10 2020 108 375 B3 beschrieben sind. Insbesondere erfolgt das tomographische Drucken grundsätzlich auf die in der DE 10 2020 108 375 B3 beschriebenen Art und Weise. Die Bilder des Datensatzes können zum Erzeugen und/oder Bereitstellen beispielsweise aus einem dreidimensionalen Datensatz (z.B. CAD-Daten), der die Form der Intraokularlinse beinhaltet, errechnet werden. Hierbei handelt es sich insbesondere um einen umgekehrten Prozess, wie er bei einer tomographischen Bildgebung eingesetzt wird. Die tomographische Bildgebung kommt beispielsweise bei einer Computertomographie zum Einsatz. Die tomographische Bildgebung kann beispielsweise von einer Radon-Transformation Gebrauch machen. Das tomographische Drucken macht sich insbesondere diesen umgekehrten Prozess zunutze, um mittels der errechneten Bilder die aushärtbare Flüssigkeit in Abhängigkeit von den in den Bildern des Datensatzes enthaltenen Lichtmustern lokal auszuhärten.

Die aushärtbare Flüssigkeit umfasst insbesondere eine Lösung mit einem gelösten Monomer und einem Fotoinitiator, welcher strahlungsabhängig eine Polymerisation des Monomers auslösen kann. Ferner können auch weitere Stoffe Bestandteil der aushärtbaren Flüssigkeit sein, wie z.B. Füllstoffe, optisch anregbare Farbstoffe oder Nanopartikel und/oder medizinische Wirkstoffe. Insbesondere ist vorgesehen, dass die aushärtbare Flüssigkeit eine vorgegebene Mindestviskosität von mindestens 100 mPa*s (cps) aufweist. Die Mindestviskosität sorgt insbesondere dafür, dass die Intraokularlinse während des tomographischen Druckens an derselben Position verbleibt. Alternativ oder zusätzlich kann auch eine Unterstützungseinrichtung vorgesehen sein, um die Intraokularlinse beim tomographischen Drucken in Position zu halten.

Als Ausgangsstoffe für die aushärtbare Flüssigkeit können insbesondere Hydroxyethylmethacrylat (HEMA), Methacrylsäuremethylester (MMA) oder 2-Ethoxyethylmethacrylat (EOEMA) verwendet werden.

Es kann vorgesehen sein, dass die Intraokularlinse eine akkommodierende Intraokularlinse ist. Ferner kann die Intraokularlinse zusätzlich einen Aktuator, ein Solarmodul und/oder einen Sensor aufweisen, die zum Einbringen in der aushärtbaren Flüssigkeit angeordnet werden, sodass die Intraokularlinse um diese Gegenstände herum gedruckt werden kann. Ferner kann die Intraokularlinse einen Optikköper und mindestens eine Haptik umfassen.

Es kann vorgesehen sein, dass die verfahrensgemäß hergestellte Intraokularlinse anschließend noch nachbearbeitet wird, beispielsweise durch Drehen, mechanisches Polieren, Laserpolieren und/oder Laserschneiden etc.

In einer Ausführungsform ist vorgesehen, dass das Deaktivieren ein Durchtränken und/oder Extrahieren der gedruckten Intraokularlinse mit und/oder in einer vorgegebenen Extraktionsflüssigkeit oder einem vorgegebenen Extraktionsgas umfasst. Die Begriffe Extraktionsflüssigkeit und Extraktionsgas sind hierbei insbesondere lediglich zur begrifflichen Abgrenzung gewählt und bezeichnen insbesondere eine Flüssigkeit oder ein Gas, welche im Rahmen des Deaktivierens eingesetzt werden. Das Deaktivieren wird hierbei insbesondere dadurch bewirkt und/oder unterstützt, dass mittels der Flüssigkeit oder des Gases mindestens ein Bestandteil aus der in dem mindestens einen Hohlraum verbleibenden aushärtbaren Flüssigkeit entfernt (extrahiert) wird. Hierzu wird die verbleibende aushärtbare Flüssigkeit nach dem tomographischen Drucken aus dem Behälter entfernt und die vorgegebene Extraktionsflüssigkeit oder das vorgegebene Extraktionsgas in den Behälter eingeleitet. Die Extraktionsflüssigkeit oder das Extraktionsgas führen zu einem Deaktivieren der aushärtbaren Flüssigkeit oder unterstützen zumindest das Deaktivieren.

In einer Ausführungsform ist vorgesehen, dass die aushärtbare Flüssigkeit ein mit Bezug auf die vorgegebene Extraktionsflüssigkeit unlösliches Monomer und einen mit Bezug auf die vorgegebene Extraktionsflüssigkeit löslichen Fotoinitiator umfasst, wobei der in dem mindestens einen Hohlraum vorhandene Fotoinitiator durch das Durchtränken und/oder Extrahieren aus dem mindestens einen Hohlraum entfernt wird. Hierdurch kann der in dem mindestens einen Hohlraum vorhandene Fotoinitiator aus diesem entfernt werden, sodass ein Aktivieren der Polymerisation in dem mindestens einen Hohlraum nicht mehr möglich ist. Beispielsweise kann vorgesehen sein, dass die aushärtbare Flüssigkeit Hydroxyethylmethacrylat (HEMA) mit einem Fotoinitiator (z.B. 1 Gewichts-% in Bezug auf das HEMA-Monomer) umfasst und hierdurch tomographisch gedruckt werden kann. Der Fotoinitiator wird hierbei derart gewählt, dass dieser sich gut in der Extraktionsflüssigkeit löst. Das Monomer wird hingegen derart gewählt, dass dieses nicht in der Extraktionsflüssigkeit gelöst werden kann. Nach dem tomographischen Drucken wird die gedruckte Intraokularlinse dann in die vorgegebene Extraktionsflüssigkeit eingebracht oder die vorgegebene Extraktionsflüssigkeit wird in den Behälter mit der darin enthaltenen gedruckten Intraokularlinse eingeleitet. Die gedruckte Intraokularlinse verbleibt dann in der vorgegebenen Extraktionsflüssigkeit für eine vorgegebene Zeitdauer. In dieser Zeitdauer wird der lösliche Fotoinitiator in der vorgegebenen Extraktionsflüssigkeit gelöst, welche durch die (permeable) Wand (z.B. Poly-HEMA) des mindestens einen Hohlraums in den mindestens einen Hohlraum eindringt und den gelösten Fotoinitiator aus dem mindestens einen Hohlraum auswäscht. Nach der vorgegebenen Zeitdauer geht der Anteil des Fotoinitiators in der verbleibenden Flüssigkeit in dem mindestens einen Hohlraum gegen Null und ein Aktivieren des Aushärtens bzw. der Polymerisation ist nicht mehr möglich.

In einer alternativen Ausführungsform ist vorgesehen, dass die aushärtbare Flüssigkeit ein mit Bezug auf die vorgegebene Extraktionsflüssigkeit lösliches Monomer umfasst, wobei das in dem mindestens einen Hohlraum vorhandene Monomer durch das Durchtränken und/oder Extrahieren aus dem mindestens einen Hohlraum entfernt wird. Prinzipiell ist das Vorgehen hierbei analog zu der voranstehend beschriebenen Ausführungsform.

Insbesondere ist in einer Ausführungsform vorgesehen, dass die vorgegebene Extraktionsflüssigkeit Wasser ist.

In einer Ausführungsform ist vorgesehen, dass die aushärtbare Flüssigkeit ein Makromonomer mit einer großen molaren Masse (insbesondere >1000 g/mol) umfasst. Somit kann der Fotoinitiator durch das Durchtränken und/oder Extrahieren aus dem mindestens einen Hohlraum entfernt werden, während das Makromonomer in dem Hohlraum verbleibt. Wird Wasser als Extraktionsflüssigkeit verwendet, so kann als wasserunlösliches Monomer beispielsweise ein Polymethylmethacrylat-(PMMA)-Makromonomer verwendet werden.

In einer Ausführungsform ist vorgesehen, dass die gedruckte Intraokularlinse mit dem mindestens einen Hohlraum im Rahmen des Deaktivierens einer kettenbrechenden Reagenz ausgesetzt wird. Es ist hierbei insbesondere vorgesehen, dass die kettenbrechende Reagenz durch die polymerisierte Außenwand des mindestens einen Hohlraums in die im mindestens einen Hohlraum vorhandene Flüssigkeit hineindiffundiert und dort ein weiteres Aktivieren der aushärtbaren Flüssigkeit verhindert. Beispiele für kettenbrechende Reagenzien sind chemische Stoffe, welche Halogene oder Halogenradikale (z.B. Brom) umfassen. Diese können sich entweder an das Endacrylat (z.B. Br2) anlagern oder sogar eine intramolekulare Zyklisierungsreaktion hervorrufen, welche beide zu einem Deaktivieren des Monomers führen.

In einer Ausführungsform ist hierbei insbesondere vorgesehen, dass die kettenbrechende Reagenz mindestens eine der folgenden ist oder umfasst: Sauerstoff oder Wasserstoff. Sauerstoff und Wasserstoff können die radikalische Kettenpolymerisation unterdrücken.

In einer Ausführungsform ist vorgesehen, dass die der kettenbrechenden Reagenz ausgesetzte gedruckte Intraokularlinse im Rahmen des Deaktivierens mit elektromagnetischer Strahlung einer vorgegebenen Wellenlänge oder eines vorgegebenen Wellenlängenbereichs bestrahlt wird. Hierdurch kann erreicht werden, dass die in dem mindestens einen Hohlraum verbleibenden Monomere aktiviert werden. Die Wahrscheinlichkeit ist hierbei hoch, dass die verbleibenden Monomere hierbei mit der vorhandenen kettenbrechenden Reagenz reagieren, sodass nur Oligomere mit einer geringen molaren Masse entstehen, welche nicht vernetzt sind und daher flüssig bleiben. Das nachfolgende Beispiel verdeutlicht das prinzipielle Vorgehen: Beispielsweise kann die aushärtbare Flüssigkeit HEMA mit einem Fotoinitiator aufweisen, der ein Absorptionsmaximum bei einer Wellenlänge von etwa 400 nm hat. HEMA weist einen Absorptionsbereich auf, der bis zu einer Wellenlänge von etwa 250 nm und darunter reicht. In einem ersten Schritt wird eine Lichtquelle mit einer Wellenlänge im Bereich von 400 nm verwendet, um in der aushärtbaren Flüssigkeit durch tomographisches Drucken das HEMA zu polymerisieren. Nach dem tomographischen Drucken wird der HEMAgefüllte mindestens eine Hohlraum der Intraokularlinse einer Umgebung mit der kettenbrechenden Reagenz ausgesetzt (z.B. Sauerstoff, Wasserstoff, Wasser). Gase können sich ungehindert in die Monomer-gefüllte Struktur hineinbewegen. Bei Flüssigkeiten hängt dies von der chemischen Struktur der Wand des mindestens einen Hohlraums ab. Anschließend wird die Intraokularlinse mit dem mindestens einen Hohlraum mit UV-Licht mit einer Wellenlänge von etwa 250 nm oder darunter bestrahlt. Dies löst die Aktivierung der verbleibenden HEMA-Monomere mit einer Acrylatgruppe aus. Wie bereits voranstehend beschrieben, reagieren diese Monomere mit großer Wahrscheinlichkeit mit der kettenbrechenden Reagenz und es werden nur HEMA-Oligomere mit geringer molarer Masse erzeugt, die nicht vernetzt sind und daher flüssig bleiben.

In einer Ausführungsform ist vorgesehen, dass die aushärtbare Flüssigkeit einen Vernetzer (Crosslinker) umfasst, der zwei oder mehr Acrylat- oder Methacrylat(end)gruppen zur Polymerisation und eine kovalent gebundene organische Molekülkette dazwischen aufweist, welche mindestens eine fotospaltbare Gruppe aufweist, welche bei Bestrahlung mit elektromagnetischer Strahlung im UV-Wellenlängenbereich gespalten wird und zu einer Spaltung der Molekülkette führt, wobei in der aushärtbaren Flüssigkeit ein Fotoinitiator verwendet wird, der bei einer Wellenlänge größer als der UV-Wellenlängenbereich aktiviert werden kann, wobei der Fotoinitiator in der aushärtbaren Flüssigkeit während des tomographischen Druckens bei einer Wellenlänge größer als der UV-Wellenlängenbereich aktiviert wird, sodass die Molekülkette durch das tomographische Drucken nicht gespalten wird, wobei die gedruckte Intraokularlinse mit dem mindestens einen Hohlraum zum Spalten der fotospaltbaren Gruppe im Rahmen des Deaktivierens mit elektromagnetischer Strahlung im UV-Wellenlängenbereich bestrahlt wird. Mögliche Crosslinker können zum Beispiel sein: Trimethylolpropantrimethacrylat, Ethylenglycoldimethacrylat, 1,4-Butandioldiacrylat, 1,4-Phenylendiacrylat. Das Verfahren ist aber nicht auf diese Beispiele beschränkt, es können grundsätzlich auch andere Crosslinker zum Einsatz kommen. Als fotospaltbare Gruppe kann beispielsweise Phenacyl verwendet werden, welches ein aromatischer Substituent ist, der ein an eine Acylgruppe angelagertes Phenyl aufweist. Neben anderen ist diese Phenacylgruppe dafür bekannt, dass eine Photodeprotektionsreaktion stattfindet, wenn eine Bestrahlung im UV-Wellenlängenbereich erfolgt, was zu einer Spaltung der molekularen Kette zwischen den Vernetzerendgruppen führt. Um diese Spaltung nicht während des tomographischen Druckens auszulösen, muss der Fotoinitiator derart gewählt werden, dass dieser bei einer größeren Wellenlänge aktiviert werden kann, insbesondere in einem Wellenlängenbereich oberhalb des UV-Wellenlängenbereichs, das heißt, insbesondere im sichtbaren Wellenlängenbereich.

In einer Ausführungsform ist vorgesehen, dass das Deaktivieren ein thermisches Aktivieren umfasst. Insbesondere ist bei dieser Ausführungsform vorgesehen, dass ein Monomer verwendet wird, das im Rahmen des offenbarten Verfahrens photochemisch ausgehärtet wird und thermisch deaktiviert wird.

Weitere Merkmale zur Ausgestaltung der Anordnung ergeben sich aus der Beschreibung von Ausgestaltungen des Verfahrens. Die Vorteile der Anordnung sind hierbei jeweils die gleichen wie bei den Ausgestaltungen des Verfahrens.

Nachfolgend wird die Erfindung anhand bevorzugter Ausführungsbeispiele unter Bezugnahme auf die Figuren näher erläutert. Hierbei zeigen:
- Fig. 1: eine schematische Darstellung von Ausführungsformen des Verfahrens;
- Fig. 2: eine schematische Darstellung zur Verdeutlichung einer Ausführungsform des Verfahrens;
- Fig. 3: eine schematische Darstellung zur Verdeutlichung von weiteren Ausführungsformen des Verfahrens;
- Fig. 4: eine schematische Darstellung einer Ausführungsform der Anordnung.

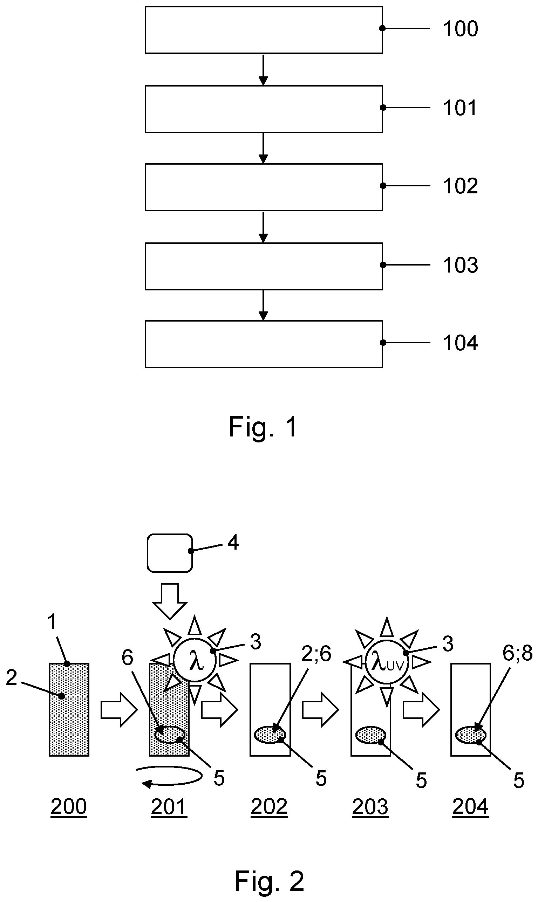

Die Fig. 1 zeigt eine schematische Darstellung von Ausführungsformen des Verfahrens. In einem Verfahrensschritt 100 wird ein für elektromagnetische Strahlung transparenter Behälter bereitgestellt. Der Behälter wird hierbei insbesondere derart gewählt, dass dieser zumindest für die in dem Verfahren verwendete elektromagnetische Strahlung, insbesondere im sichtbaren und UV-Wellenlängenbereich, transparent ist.

In einem Verfahrensschritt 101 wird eine mittels elektromagnetischer Strahlung aushärtbare Flüssigkeit in dem Behälter bereitgestellt. Insbesondere handelt es sich hierbei um eine Flüssigkeit, in der Monomere und ein Fotoinitiator enthalten sind, wobei die Monomere durch Aktivieren des Fotoinitiators polymerisiert werden können. Die aushärtbare Flüssigkeit wird beispielsweise in den Behälter eingefüllt.

In einem Verfahrensschritt 102 wird ein Datensatz aus Bildern einer Intraokularlinse mit mindestens einem Hohlraum erzeugt und/oder bereitgestellt, wobei die Bilder Projektionen der Intraokularlinse mit dem mindestens einen Hohlraum aus unterschiedlichen Richtungen beinhalten. Die Bilder werden hierbei insbesondere ausgehend von dreidimensionalen Daten (z.B. eines CAD-Modells der Intraokularlinse mit dem mindestens einen Hohlraum) erzeugt, indem Projektionen von elektromagnetischer Strahlung aus unterschiedlichen Richtungen durch die dreidimensionale Intraokularlinse mit dem mindestens einen Hohlraum hindurch berechnet werden, welche eine Absorption des Lichtes durch das Material der Intraokularlinse mit dem mindestens einen Hohlraum beschreiben. Hierbei handelt es sich um einen umgekehrten Prozess, wie er bei einer tomographischen Bildgebung eingesetzt wird. Die tomographische Bildgebung kommt beispielsweise bei einer Computertomographie zum Einsatz. Die tomographische Bildgebung kann beispielsweise von einer Radon-Transformation bzw. deren Rücktransformation Gebrauch machen.

In einem Verfahrensschritt 103 wird die aushärtbare Flüssigkeit mittels elektromagnetischer Strahlung ausgehend von dem erzeugten und/oder bereitgestellten Datensatz zum Ausbilden der Intraokularlinse mit dem mindestens einen Hohlraum tomographisch gedruckt. Hierbei erfolgt eine Bestrahlung der aushärtbaren Flüssigkeit mit elektromagnetischer Strahlung ausgehend von den Bildern aus dem erzeugten und/oder bereitgestellten Datensatz aus unterschiedlichen Richtungen, wie dies beispielsweise in der DE 10 2020 108 375 B3 beschrieben ist. Anders ausgedrückt werden die Bilder aus einer jeweils hiermit korrespondierenden Richtung mittels elektromagnetischer Strahlung in die aushärtbare Flüssigkeit projiziert. Hierdurch wird die aushärtbare Flüssigkeit an den jeweils gewünschten Stellen polymerisiert und härtet aus, wodurch die Intraokularlinse mit dem mindestens einen Hohlraum ausgebildet wird.

In einem Verfahrensschritt 104 wird die Fähigkeit zum Aushärten der aushärtbaren Flüssigkeit in dem mindestens einen Hohlraum deaktiviert. Ein weiteres Aushärten der aushärtbaren Flüssigkeit in dem mindestens einen Hohlraum ist dann nicht mehr möglich. Insbesondere bleiben die in der Flüssigkeit vorhandenen Monomere flüssig und können nicht mehr polymerisiert werden.

Es kann im Verfahrensschritt 104 vorgesehen sein, dass das Deaktivieren ein Durchtränken und/oder Extrahieren der gedruckten Intraokularlinse mit und/oder in einer vorgegebenen Extraktionsflüssigkeit oder einem vorgegebenen Extraktionsgas umfasst. Insbesondere können hierdurch das eingesetzte Monomer und/oder der eingesetzte Fotoinitiator durch Tränken in einer Lösung (z.B. einem Dien und Reaktion des (Meth)Acrylats über Diels-Alder-Reaktion) oder bei Veranschlagung mit einem geeigneten Gas (z.B. Wasserstoff/Sauerstoff, Deaktivierung der Acrylatfunktion/sofortiger Kettenabbruch) deaktiviert werden. Das Extrahieren kann zum Beispiel mit einer hydrophoben Extraktionsflüssigkeit (z.B. Hexan) erfolgen. Hierdurch lassen sich Fotoinitiatoren, nicht polymerisierte Monomere sowie nicht vollständig polymerisierte niedermolekulare Oligomere aus dem Polymer entfernen und die weitere Polymerisation somit effektiv beenden.

Es kann weiterbildend vorgesehen sein, dass die aushärtbare Flüssigkeit ein mit Bezug auf die vorgegebene Extraktionsflüssigkeit unlösliches Monomer und einen mit Bezug auf die vorgegebene Extraktionsflüssigkeit löslichen Fotoinitiator umfasst, wobei der in dem mindestens einen Hohlraum vorhandene Fotoinitiator durch das Durchtränken und/oder Extrahieren aus dem mindestens einen Hohlraum entfernt wird. Beispielsweise kann ein wasserlöslicher Fotoinitiator verwendet werden (z.B. Kaliumpersulfat oder 4,4'-Azobis(4-cyanovaleric-acid, ACVA), welcher aus dem Hohlraum mit darin verbleibendem nicht reagiertem Monomer entfernt wird. Das Monomer (z.B. Octadecylmethacrylat) ist nicht wasserlöslich und verbleibt somit im Hohlraum. Auf diese Weise kann insbesondere verhindert werden, dass das verbleibende Monomer im Hohlraum ausgehärtet wird.

Alternativ kann weiterbildend vorgesehen sein, dass die aushärtbare Flüssigkeit ein mit Bezug auf die vorgegebene Extraktionsflüssigkeit lösliches Monomer umfasst, wobei das in dem mindestens einen Hohlraum vorhandene Monomer durch das Durchtränken und/oder Extrahieren aus dem mindestens einen Hohlraum entfernt wird. Beispielsweise kann vorgesehen sein, dass sowohl der verwendete Fotoinitiator (z.B. ACVA) als auch das verwendete Monomer (z.B. Hydroxymethylmethacrylat) wasserlöslich sind. Mittels der Extraktionsflüssigkeit (im Beispiel Wasser) werden sowohl der Fotoinitiator als auch das Monomer aus dem Hohlraum entfernt und der Hohlraum mit der Extraktionsflüssigkeit (im Beispiel Wasser) aufgefüllt. Grundsätzlich können anstatt Wasser auch andere Lösungsmittel eingesetzt werden, wobei der Fotoinitiator und das Monomer dann entsprechend ausgewählt werden.

Insbesondere kann vorgesehen sein, dass die vorgegebene Extraktionsflüssigkeit Wasser ist. In der ersten Alternative ist das Monomer dann wasserunlöslich und der Fotoinitiator wasserlöslich. In der zweiten Alternative ist das Monomer dann wasserlöslich.

Es kann vorgesehen sein, dass die aushärtbare Flüssigkeit ein Makromonomer mit einer großen molaren Masse (insbesondere >1000 g/mol) umfasst. Das Makromonomer kann beispielsweise PMMA sein. Insbesondere wird das Makromonomer mit einem sekundären Monomer kombiniert. Hierzu gibt es eine Vielzahl an möglichen Kombinationen.

In der nachfolgenden Tabelle 1 sind Beispiele für Makromonomere und sekundäre Monomere aufgeführt, welche repräsentativ, aber nicht einschränkend sind. Beispielsweise kann auch eines der PMMA-Präpolymere mit einem oder mehreren der sekundären Monomere in der Tabelle verbunden werden. Es ist auch möglich, verschiedene Präpolymere zu mischen. Z.B. PMMA-Präpolymere verschiedener Längen, oder Präpolymere aus PMMA mit Präpolymeren der sekundären Monomere. Weiterhin kann ein Mischungsanteil mit jedem möglichen Verhältnis zueinander variiert werden, um gewünschte Eigenschaften des resultierenden Copolymers zu erhalten.

**Tabelle 1: Beispiele für Formulierungen aus PMMA-Makromonomeren und verschiedenen Kandidaten für Copolymere. Hierbei kann jedes der Makromonomere (Präpolymere) mit jedem der sekundären Monomere verknüpft werden. Auch die Mengenanteile sind variabel.**

| **PMMA-Makromonomer** | **Sekundäres Monomer** | **Crosslinker** |
|---|---|---|
| (C₅O₂H₈)₅₀₋₁₀₀ | Hydroxyethylmethacrylat | |
| (C₅O₂H₈)₁₀₀₋₂₀₀ | Ethoxyethylmethacrylat | Trimethylopropantrimethacrylat |
| (C₅O₂H₈)₅₀₀₋₇₀₀ | Ethylenglycoldimethacrylat | |
| (C₅O₂H₈)₈₀₀₋₁₀₀₀ | Butylacrylat | |
| (C₅O₂H₈)₁₀₀₀₋₁₅₀₀ | Ethylmethacrylat | |

Es kann in Verfahrensschritt 104 vorgesehen sein, dass die gedruckte Intraokularlinse mit dem mindestens einen Hohlraum im Rahmen des Deaktivierens einer kettenbrechenden Reagenz ausgesetzt wird.

Insbesondere kann vorgesehen sein, dass die kettenbrechende Reagenz mindestens eine der folgenden ist oder umfasst: Sauerstoff, Wasserstoff, Wasser.

Ferner kann vorgesehen sein, dass als kettenbrechende Reagenz ein chemischer Stoff verwendet wird, der Halogene oder Halogenradikale (z.B. Brom) umfasst. Diese können sich dann entweder an die Endacrylate (z.B. Br₂) anlagern oder sogar eine intramolekulare Zyklisierungsreaktion starten, was beides zu einer Deaktivierung des Monomers führt, sodass im Anschluss keine weitere Aushärtung bzw. Polymerisation möglich ist.

Es kann in Verfahrensschritt 104 ferner vorgesehen sein, dass die der kettenbrechenden Reagenz ausgesetzte gedruckte Intraokularlinse im Rahmen des Deaktivierens mit elektromagnetischer Strahlung einer vorgegebenen Wellenlänge oder eines vorgegebenen Wellenlängenbereichs bestrahlt wird. Hierdurch werden in der aushärtbaren Flüssigkeit enthaltene Monomere aktiviert, wobei diese mit der kettenbrechenden Reagenz (z.B. Sauerstoff, Wasserstoff oder Wasser) reagieren und hierbei lediglich Oligomere mit einer kleinen molaren Masse entstehen, welche nicht vernetzt sind und daher flüssig bleiben. Die vorgegebene Wellenlänge oder der vorgegebene Wellenlängenbereich liegt hierbei insbesondere im UV-Bereich, das heißt, insbesondere bei Wellenlängen von 250 nm und darunter.

Es kann vorgesehen sein, dass die aushärtbare Flüssigkeit einen Vernetzer (Crosslinker) umfasst, der zwei oder mehr Acrylat- oder Methacrylat(end)gruppen zur Polymerisation und eine kovalent gebundene organische Molekülkette dazwischen aufweist, welche mindestens eine fotospaltbare Gruppe aufweist, welche bei Bestrahlung mit elektromagnetischer Strahlung im UV-Wellenlängenbereich gespalten wird und zu einer Spaltung der Molekülkette führt, wobei in der aushärtbaren Flüssigkeit ein Fotoinitiator verwendet wird, der bei einer Wellenlänge größer als der UV-Wellenlängenbereich aktiviert werden kann, wobei der Fotoinitiator in der aushärtbaren Flüssigkeit während des tomographischen Druckens in Verfahrensschritt 103 bei einer Wellenlänge größer als der UV-Wellenlängenbereich aktiviert wird, sodass die Molekülkette durch das tomographische Drucken nicht gespalten wird, wobei die gedruckte Intraokularlinse mit dem mindestens einen Hohlraum zum Spalten der fotospaltbaren Gruppe im Rahmen des Deaktivierens mit elektromagnetischer Strahlung in Verfahrensschritt 104 im UV-Wellenlängenbereich bestrahlt wird.

Es kann in Verfahrensschritt 104 vorgesehen sein, dass das Deaktivieren ein thermisches Aktivieren umfasst.

Die Fig. 2 zeigt eine schematische Darstellung zur Verdeutlichung einer Ausführungsform des Verfahrens. Der Grundsätzliche Ablauf ist hierbei der gleiche wie dieser bereits mit Bezug auf die Fig. 1 beschrieben wurde.

In einem für elektromagnetische Strahlung transparenten Behälter 1 wird in einem Verfahrensschritt 200 eine mittels elektromagnetischer Strahlung aushärtbare Flüssigkeit 2 bereitgestellt. Die aushärtbare Flüssigkeit 2 umfasst einen Vernetzer (Crosslinker), der zwei oder mehr Acrylat- oder Methacrylat(end)gruppen zur Polymerisation und eine kovalent gebundene organische Molekülkette dazwischen aufweist, welche mindestens eine fotospaltbare Gruppe aufweist. Die fotospaltbare Gruppe wird bei Bestrahlung mit elektromagnetischer Strahlung im UV-Wellenlängenbereich gespalten, was zu einer Spaltung der Molekülkette führt. In der aushärtbaren Flüssigkeit 2 wird ferner ein Fotoinitiator verwendet, der bei einer Wellenlänge größer als der UV-Wellenlängenbereich aktiviert werden kann.

In einem Verfahrensschritt 201 wird die aushärtbare Flüssigkeit 2 mittels elektromagnetischer Strahlung 3 ausgehend von einem erzeugten und/oder bereitgestellten Datensatz 4 zum Ausbilden einer Intraokularlinse 5 mit dem mindestens einen Hohlraum 6 tomographisch gedruckt. Der Datensatz 4 umfasst Bilder der Intraokularlinse 5 mit dem mindestens einen Hohlraum 6, wobei die Bilder Projektionen der Intraokularlinse 5 mit dem mindestens einen Hohlraum 6 aus unterschiedlichen Richtungen beinhalten. Der Datensatz 4 kann beispielsweise aus einem CAD-Modell der Intraokularlinse 5 mit dem mindestens einen Hohlraum 6 berechnet werden, indem aus unterschiedlichen Richtungen Projektionen durch das CAD-Modell hindurch berechnet werden, welche eine orts- und materialabhängige Schwächung der elektromagnetischen Strahlung abbilden. Das prinzipielle Vorgehen ist beispielsweise in der DE 10 2020 108 375 B3 beschrieben.

Es ist beim tomographischen Drucken in Verfahrensschritt 201 vorgesehen, dass der Fotoinitiator in der aushärtbaren Flüssigkeit 2 bei einer Wellenlänge λ größer als der UV-Wellenlängenbereich λ_{UV} aktiviert wird, sodass die kovalent gebundene organische Molekülkette durch das tomographische Drucken nicht gespalten wird.

Nach dem tomographischen Drucken wird die aushärtbare Flüssigkeit 2 in einem Verfahrensschritt 202 aus dem Behälter 1 entfernt. Die gedruckte Intraokularlinse 5 verbleibt in dem Behälter 1, wobei der mindestens eine Hohlraum 6 durch eine äußere Hülle der Intraokularlinse 5 abgeschlossen ist und weiterhin die aushärtbare Flüssigkeit 2 beinhaltet.

In einem Verfahrensschritt 203 wird die gedruckte Intraokularlinse 5 mit dem mindestens einen Hohlraum 6 zum Spalten der fotospaltbaren Gruppe im Rahmen des Deaktivierens mit elektromagnetischer Strahlung 3 im UV-Wellenlängenbereich λ_{UV} bestrahlt. Dies führt zu einer Spaltung der Molekülkette zwischen den Vernetzerendgruppen. Hierdurch kann die in dem mindestens einen Hohlraum 6 verbleibende Flüssigkeit deaktiviert werden, sodass in einem Verfahrensschritt 204 eine Intraokularlinse 5 mit einer deaktivierten Flüssigkeit 8 bereitgestellt werden kann. Ein Aushärten der deaktivierten Flüssigkeit 8 kann dann nicht mehr mittels elektromagnetischer Strahlung 3 gestartet werden, sodass der flüssige Zustand beibehalten wird.

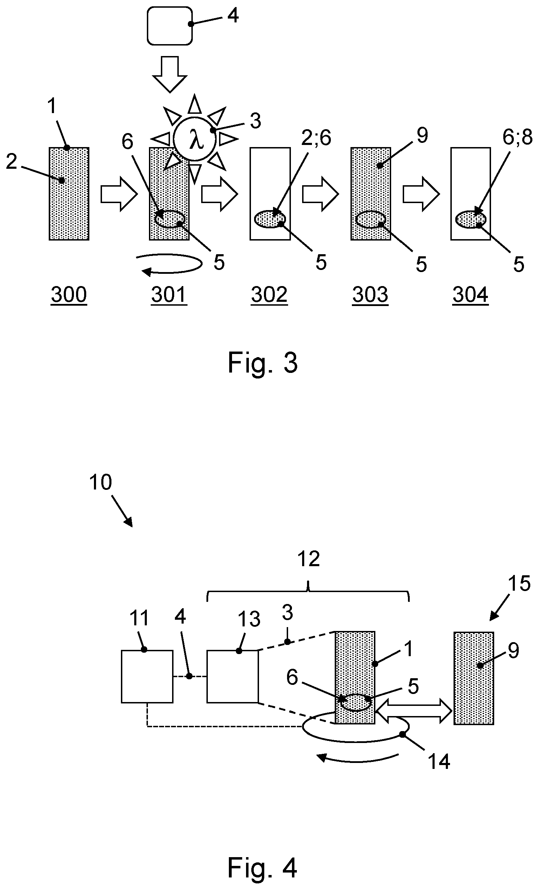

Die Fig. 3 zeigt eine schematische Darstellung zur Verdeutlichung von weiteren Ausführungsformen des Verfahrens. Der Grundsätzliche Ablauf ist hierbei der gleiche wie dieser bereits mit Bezug auf die Fig. 1 beschrieben wurde.

In einem für elektromagnetische Strahlung transparenten Behälter 1 wird in einem Verfahrensschritt 300 eine mittels elektromagnetischer Strahlung aushärtbare Flüssigkeit 2 bereitgestellt. Es ist vorgesehen, dass die aushärtbare Flüssigkeit 2 ein mit Bezug auf eine vorgegebene Extraktionsflüssigkeit unlösliches Monomer, beispielsweise ein Makromonomer mit einer großen molaren Masse (z.B. ein PMMA-Makromonomer), und einen mit Bezug auf die vorgegebene Extraktionsflüssigkeit löslichen Fotoinitiator umfasst. Der Anteil des Fotoinitiators beträgt beispielsweise 1-Gewichts-% des Monomers in der aushärtbaren Flüssigkeit 2. Beispiele sind in der nachfolgenden Tabelle 2 angegeben.

**Tabelle 2: Beispiele für Fotoinitiatoren und mögliche Extraktionsmittel. Jeder der Initiatoren kann mit jedem der genannten Extraktionsmittel extrahiert werden.**

| **Fotoinitiator** | **Extraktionsmittel** |
|---|---|
| 2,2-Dimethoxy-1,2-diphenylethan-1-on | Hexan |
| 2-Hydroxy-2-methyl-1-phenylpropanon | Cyclohexan |
| 1-hydroxy-cyclohexylphenylketon | Isopropanol |
| 1,2-Diphenyl-2,2-dimethoxyethanon (Irgacure 651) | DMSO |
| (2,4,6-trimethylbenzoyl)phosphinoxid (Irgacure 819) | Ethanol, Methanol |
| Benzophenon | Benzol |
| Isopropylthioxanthon | DMSO |

In einem Verfahrensschritt 301 wird die aushärtbare Flüssigkeit 2 mittels elektromagnetischer Strahlung 3 ausgehend von einem erzeugten und/oder bereitgestellten Datensatz 4 zum Ausbilden einer Intraokularlinse 5 mit dem mindestens einen Hohlraum 6 tomographisch gedruckt. Der Datensatz 4 kann hierbei auf die gleiche Weise erzeugt und/oder bereitgestellt werden, wie dies bereits für das in der Fig. 2 gezeigte Ausführungsbeispiel beschrieben wurde.

In einem Verfahrensschritt 302 wird die aushärtbare Flüssigkeit 2 aus dem Behälter 1 entfernt. Die gedruckte Intraokularlinse 5 verbleibt in dem Behälter 1, wobei der mindestens eine Hohlraum 6 durch eine äußere Hülle der Intraokularlinse 5 abgeschlossen ist und weiterhin die aushärtbare Flüssigkeit 2 beinhaltet.

In einem Verfahrensschritt 303 wird die vorgegebene Extraktionsflüssigkeit 9 in den Behälter 1 eingebracht. Die gedruckte Intraokularlinse 5 mit dem mindestens einen Hohlraum 6 wird hierdurch mit der vorgegebenen Extraktionsflüssigkeit 9 durchtränkt. Der in dem mindestens einen Hohlraum 6 vorhandene Fotoinitiator wird durch das Durchtränken (und/oder Extrahieren) aus dem mindestens einen Hohlraum 6 entfernt. Hierdurch kann die in dem mindestens einen Hohlraum 6 verbleibende Flüssigkeit deaktiviert werden, sodass in einem Verfahrensschritt 304 eine Intraokularlinse 5 mit einer deaktivierten Flüssigkeit 8 bereitgestellt werden kann. Ein Aushärten der deaktivierten Flüssigkeit 8 kann dann nicht mehr mittels elektromagnetischer Strahlung 3 gestartet werden, sodass der flüssige Zustand beibehalten wird.

Ist die vorgegebene Extraktionsflüssigkeit 9 beispielsweise Wasser, so werden ein wasserunlösliches Monomer und ein wasserlöslicher Fotoinitiator verwendet.

In einer alternativen Ausführungsform ist vorgesehen, dass die aushärtbare Flüssigkeit 2 ein mit Bezug auf die vorgegebene Extraktionsflüssigkeit 9 lösliches Monomer umfasst, wobei das in dem mindestens einen Hohlraum 6 vorhandene Monomer durch das Durchtränken (und/oder Extrahieren) aus dem mindestens einen Hohlraum 6 entfernt wird. Im vorgenannten Beispiel, bei dem die vorgegebene Extraktionsflüssigkeit 9 Wasser ist, wird ein wasserlösliches Monomer verwendet. Beispielsweise kann ein Hydroxyethylmethacrylat-(HEMA)-Monomer verwendet werden. Das HEMA-Monomer ist wasserlöslich und das polymerisierte HEMA der Hülle der Intraokularlinse 5 ist wasserdurchlässig, sodass das HEMA-Monomer aus dem mindestens einen Hohlraum 6 durch das Durchtränken (und/oder Extrahieren) entfernt werden kann.

Die Fig. 4 zeigt eine schematische Darstellung einer Ausführungsform der Anordnung 10 zum Herstellen einer Intraokularlinse 5. Die Anordnung 10 umfasst einen für elektromagnetische Strahlung 3 transparenten Behälter 1, eine Datenverarbeitungseinrichtung 11, die eingerichtet ist zum Erzeugen und/oder Bereitstellen eines Datensatzes 4 aus Bildern einer Intraokularlinse 5 mit mindestens einem Hohlraum 6, wobei die Bilder Projektionen der Intraokularlinse 5 mit dem mindestens einen Hohlraum 6 aus unterschiedlichen Richtungen beinhalten.

Die Anordnung 10 umfasst ferner eine tomographische Druckeinrichtung 12, die eingerichtet ist zum tomographischen Drucken einer in dem Behälter 1 bereitgestellten aushärtbaren Flüssigkeit 2 mittels elektromagnetischer Strahlung 3 ausgehend von dem erzeugten und/oder bereitgestellten Datensatz 4 zum Ausbilden der Intraokularlinse 5 mit dem mindestens einen Hohlraum 6. Die tomographische Druckeinrichtung 12 umfasst insbesondere eine Bestrahlungseinrichtung 13, welche mittels der elektromagnetischen Strahlung 3 die einzelnen Bilder durch den transparenten Behälter 1 hindurch in die aushärtbare Flüssigkeit 2 projiziert und hierdurch ortsabhängig ein Aushärten bewirkt. Ferner umfasst die Druckeinrichtung 12 beispielsweise einen Drehtisch 14, mit dem eine Bestrahlungsrichtung geändert werden kann, sodass die aushärtbare Flüssigkeit 2 aus unterschiedlichen Richtungen bestrahlt werden kann (z.B. mit Einstrahlwinkeln zwischen 0 und 360° mit Bezug auf eine Drehachse des Drehtisches 14). Es können auch mehrere Bestrahlungseinrichtungen 13 vorgesehen sein, die jeweils elektromagnetische Strahlung 3 gemäß des jeweiligen Bildes des Datensatzes 4 aus verschiedenen Richtungen gleichzeitig in die aushärtbare Flüssigkeit 2 einstrahlen.

Weiter umfasst die Anordnung 10 mindestens ein Deaktivierungsmittel 15, das eingerichtet ist zum Deaktivieren der Fähigkeit zum Aushärten der aushärtbaren Flüssigkeit 2 in dem mindestens einen Hohlraum 6 oder das Deaktivieren vorzubereiten und/oder zu unterstützen. Beispielsweise kann vorgesehen sein, dass als Deaktivierungsmittel 15 ein Flüssigkeitsreservoir vorgesehen ist, das dazu eingerichtet ist, eine vorgegebene Extraktionsflüssigkeit 9 in den Behälter 1 einzubringen und wieder aus diesem zu entfernen (in der Fig. 4 lediglich schematisch angedeutet), um hierdurch (mit oder ohne zusätzliche elektromagnetische Bestrahlung) das Deaktivieren zu bewirken.

### Bezugszeichenliste

- 1: transparenter Behälter
- 2: aushärtbare Flüssigkeit
- 3: elektromagnetische Strahlung
- 4: Datensatz
- 5: Intraokularlinse
- 6: Hohlraum
- 8: deaktivierte Flüssigkeit
- 9: Extraktionsflüssigkeit
- 10: Anordnung
- 11: Datenverarbeitungseinrichtung
- 12: tomographische Druckeinrichtung
- 13: Bestrahlungseinrichtung
- 14: Drehtisch
- 15: Deaktivierungsmittel
- 100-104: Verfahrensschritte
- 200-204: Verfahrensschritte
- 300-304: Verfahrensschritte
- λ: Wellenlänge (>λ_{UV})
- λ_{UV}: Wellenlänge (UV-Bereich)

## Patentansprüche

1. Verfahren zum Herstellen einer Intraokularlinse (5), umfassend:
Bereitstellen eines für elektromagnetische Strahlung (3) transparenten Behälters (1), Bereitstellen einer mittels elektromagnetischer Strahlung (3) aushärtbaren Flüssigkeit (2) in dem Behälter (1),
Erzeugen und/oder Bereitstellen eines Datensatzes (4) aus Bildern einer Intraokularlinse (5) mit mindestens einem Hohlraum (6), wobei die Bilder Projektionen der Intraokularlinse (5) mit dem mindestens einen Hohlraum (6) aus unterschiedlichen Richtungen beinhalten,
tomographisches Drucken der aushärtbaren Flüssigkeit (2) mittels elektromagnetischer Strahlung (3) ausgehend von dem erzeugten und/oder bereitgestellten Datensatz (4) zum Ausbilden der Intraokularlinse (5) mit dem mindestens einen Hohlraum (6),
**dadurch gekennzeichnet, dass** das Verfahren umfasst:Deaktivieren der Fähigkeit zum Aushärten der aushärtbaren Flüssigkeit (2) in dem mindestens einen Hohlraum (6).

2. Verfahren nach Anspruch 1, **dadurch gekennzeichnet, dass** das Deaktivieren ein Durchtränken und/oder Extrahieren der gedruckten Intraokularlinse (5) mit und/oder in einer vorgegebenen Extraktionsflüssigkeit (9) oder einem vorgegebenen Extraktionsgas umfasst.

3. Verfahren nach Anspruch 2, **dadurch gekennzeichnet, dass** die aushärtbare Flüssigkeit (2) ein mit Bezug auf die vorgegebene Extraktionsflüssigkeit (9) unlösliches Monomer und einen mit Bezug auf die vorgegebene Extraktionsflüssigkeit (9) löslichen Fotoinitiator umfasst, wobei der in dem mindestens einen Hohlraum (6) vorhandene Fotoinitiator durch das Durchtränken und/oder Extrahieren aus dem mindestens einen Hohlraum (6) entfernt wird.

4. Verfahren nach Anspruch 2, **dadurch gekennzeichnet, dass** die aushärtbare Flüssigkeit (2) ein mit Bezug auf die vorgegebene Extraktionsflüssigkeit (9) lösliches Monomer umfasst, wobei das in dem mindestens einen Hohlraum (6) vorhandene Monomer durch das Durchtränken und/oder Extrahieren aus dem mindestens einen Hohlraum (6) entfernt wird.

5. Verfahren nach einem der Ansprüche 2 bis 4, **dadurch gekennzeichnet, dass** die vorgegebene Extraktionsflüssigkeit (9) Wasser ist.

6. Verfahren nach einem der vorangegangenen Ansprüche, **dadurch gekennzeichnet, dass** die aushärtbare Flüssigkeit (2) ein Makromonomer mit einer großen molaren Masse umfasst.

7. Verfahren nach einem der vorangegangenen Ansprüche, **dadurch gekennzeichnet, dass** die gedruckte Intraokularlinse (5) mit dem mindestens einen Hohlraum (6) im Rahmen des Deaktivierens einer kettenbrechenden Reagenz ausgesetzt wird.

8. Verfahren nach Anspruch 6 oder 7, **dadurch gekennzeichnet, dass** die der kettenbrechenden Reagenz ausgesetzte gedruckte Intraokularlinse (5) im Rahmen des Deaktivierens mit elektromagnetischer Strahlung (3) einer vorgegebenen Wellenlänge oder eines vorgegebenen Wellenlängenbereichs bestrahlt wird.

9. Verfahren nach einem der vorangegangenen Ansprüche, **dadurch gekennzeichnet, dass** die aushärtbare Flüssigkeit (2) einen Vernetzer (Crosslinker) umfasst, der zwei oder mehr Acrylat- oder Methacrylat(end)gruppen zur Polymerisation und eine kovalent gebundene organische Molekülkette dazwischen aufweist, welche mindestens eine fotospaltbare Gruppe aufweist, welche bei Bestrahlung mit elektromagnetischer Strahlung im UV-Wellenlängenbereich gespalten wird und zu einer Spaltung der Molekülkette führt, wobei in der aushärtbaren Flüssigkeit (2) ein Fotoinitiator verwendet wird, der bei einer Wellenlänge größer als der UV-Wellenlängenbereich aktiviert werden kann, wobei der Fotoinitiator in der aushärtbaren Flüssigkeit (2) während des tomographischen Druckens bei einer Wellenlänge größer als der UV-Wellenlängenbereich aktiviert wird, sodass die Molekülkette durch das tomographische Drucken nicht gespalten wird, wobei die gedruckte Intraokularlinse (5) mit dem mindestens einen Hohlraum (6) zum Spalten der fotospaltbaren Gruppe im Rahmen des Deaktivierens mit elektromagnetischer Strahlung (3) im UV-Wellenlängenbereich bestrahlt wird.

10. Anordnung (10) zum Herstellen einer Intraokularlinse (5), umfassend:
einen für elektromagnetische Strahlung (3) transparenten Behälter (2);
eine Datenverarbeitungseinrichtung (11), die eingerichtet ist zum Erzeugen und/oder Bereitstellen eines Datensatzes (4) aus Bildern einer Intraokularlinse (5) mit mindestens einem Hohlraum (6), wobei die Bilder Projektionen der Intraokularlinse (5) mit dem mindestens einen Hohlraum (6) aus unterschiedlichen Richtungen beinhalten;
eine tomographische Druckeinrichtung (12), die eingerichtet ist zum tomographischen Drucken einer in dem Behälter (1) bereitgestellten aushärtbaren Flüssigkeit (2) mittels elektromagnetischer Strahlung (3) ausgehend von dem erzeugten und/oder bereitgestellten Datensatz (4) zum Ausbilden der Intraokularlinse (5) mit dem mindestens einen Hohlraum (6); und **dadurch gekennzeichnet, dass** die Anordnung umfasst:
mindestens ein Deaktivierungsmittel (15), das eingerichtet ist zum Deaktivieren der Fähigkeit zum Aushärten der aushärtbaren Flüssigkeit (2) in dem mindestens einen Hohlraum (6) oder das Deaktivieren vorzubereiten und/oder zu unterstützen.

## Claims

1. Method of producing an intraocular lens (5), comprising:
providing a vessel (1) transparent to electromagnetic radiation (3),
providing a liquid (2) curable by electromagnetic radiation (3) in the vessel (1),
creating and/or providing a dataset (4) of images of an intraocular lens (5) with at least one cavity (6), the images comprising projections of the intraocular lens (5) with the at least one cavity (6) from different directions,
tomographically printing the curable liquid (2) by means of electromagnetic radiation (3) proceeding from the dataset (4) created and/or provided to form the intraocular lens (5) with the at least one cavity (6),
**characterized in that** the method comprises: deactivating the curability of the curable liquid (2) in the at least one cavity (6).

2. Method according to Claim 1, **characterized in that** the deactivating comprises impregnating and/or extracting the printed intraocular lens (5) with and/or in a defined extraction liquid (9) or a defined extraction gas.

3. Method according to Claim 2, **characterized in that** the curable liquid (2) comprises a monomer that is insoluble with respect to the defined extraction liquid (9) and a photoinitiator that is soluble with respect to the defined extraction liquid (9), where the photoinitiator present in the at least one cavity (6) is removed from the at least one cavity (6) by the impregnating and/or extracting operation.

4. Method according to Claim 2, **characterized in that** the curable liquid (2) comprises a monomer that is soluble with respect to the defined extraction liquid (9), where the monomer present in the at least one cavity (6) is removed from the at least one cavity (6) by the impregnating and/or extracting operation.

5. Method according to any of Claims 2 to 4, **characterized in that** the defined extraction liquid (9) is water.

6. Method according to any of the preceding claims, **characterized in that** the curable liquid (2) comprises a macromonomer having a large molar mass.

7. Method according to any of the preceding claims, **characterized in that** the printed intraocular lens (5) with the at least one cavity (6) is exposed to a chain-terminating reagent in the deactivating operation.

8. Method according to Claim 6 or 7, **characterized in that** the printed intraocular lens (5) exposed to the chain-terminating reagent in the deactivating operation is irradiated with electromagnetic radiation (3) of a defined wavelength or a defined wavelength range.

9. Method according to any of the preceding claims, **characterized in that** the curable liquid (2) comprises a crosslinker having two or more acrylate or methacrylate (end) groups for polymerization and a covalently bonded organic molecular chain in between that has at least one photocleavable group which is cleaved on irradiation with electromagnetic radiation in the UV wavelength range and leads to cleavage of the molecular chain, where a photoinitiator that is used in the curable liquid (2) can be activated at a wavelength greater than the UV wavelength range, where the photoinitiator in the curable liquid (2) is activated during the tomographic printing at a wavelength greater than the UV wavelength range, such that the molecular chain is not cleaved by the tomographic printing, where the printed intraocular lens (5) with the at least one cavity (6), for cleavage of the photocleavable group, is irradiated with electromagnetic radiation (3) in the UV wavelength range in the deactivating operation.

10. Assembly (10) for producing an intraocular lens (5), comprising:
a vessel (2) transparent to electromagnetic radiation (3);
a data processing device (11) set up to create and/or provide a dataset (4) of images of an intraocular lens (5) with at least one cavity (6), the images comprising projections of the intraocular lens (5) with the at least one cavity (6) from different directions;
a tomographic printing device (12) set up for tomographic printing of a curable liquid (2) provided in the vessel (1) by means of electromagnetic radiation (3) proceeding from the dataset (4) created and/or provided to form the intraocular lens (5) with the at least one cavity (6); and **characterized in that** the assembly comprises:
at least one deactivating means (15) set up to deactivate the curability of the curable liquid (2) in the at least one cavity (6) or to prepare for or assist the deactivating operation.

## Revendications

1. Procédé de fabrication d'une lentille intraoculaire (5), comprenant :
la fourniture d'un récipient (1) transparent au rayonnement électromagnétique (3),
la fourniture d'un liquide (2) durcissable par un rayonnement électromagnétique (3) dans le récipient (1),
la génération et/ou la fourniture d'un ensemble de données (4) à partir d'images d'une lentille intraoculaire (5) présentant au moins une cavité (6), les images contenant des projections de la lentille intraoculaire (5) présentant l'au moins une cavité (6) depuis des directions différentes,
l'impression tomographique du liquide durcissable (2) au moyen du rayonnement électromagnétique (3) à partir de l'ensemble de données (4) généré et/ou fourni pour former la lentille intraoculaire (5) présentant l'au moins une cavité (6),
**caractérisé en ce que** le procédé comprend : la désactivation de la capacité de durcissement du liquide durcissable (2) dans l'au moins une cavité (6).

2. Procédé selon la revendication 1, **caractérisé en ce que** la désactivation comprend une imprégnation et/ou une extraction de la lentille intraoculaire (5) imprimée avec et/ou dans un liquide d'extraction prédéterminé (9) ou un gaz d'extraction prédéterminé.

3. Procédé selon la revendication 2, **caractérisé en ce que** le liquide durcissable (2) comprend un monomère insoluble en ce qui concerne le liquide d'extraction prédéterminé (9) et un photoinitiateur soluble en ce qui concerne le liquide d'extraction prédéterminé (9), le photoinitiateur présent dans l'au moins une cavité (6) étant éliminé par imprégnation et/ou extraction hors de l'au moins une cavité (6).

4. Procédé selon la revendication 2, **caractérisé en ce que** le liquide durcissable (2) comprend un monomère soluble en ce qui concerne le liquide d'extraction prédéterminé (9), dans lequel le monomère présent dans l'au moins une cavité (6) est éliminé par imprégnation et/ou extraction hors de l'au moins une cavité (6).

5. Procédé selon l'une quelconque des revendications 2 à 4, **caractérisé en ce que** le liquide d'extraction prédéterminé (9) est l'eau.

6. Procédé selon l'une quelconque des revendications précédentes, **caractérisé en ce que** le liquide durcissable (2) comprend un macromonomère ayant une masse molaire élevée.

7. Procédé selon l'une quelconque des revendications précédentes, **caractérisé en ce que** la lentille intraoculaire (5) imprimée comportant l'au moins une cavité (6) est exposée, dans le cadre de la désactivation, à un réactif de rupture de chaîne.

8. Procédé selon la revendication 6 ou 7, **caractérisé en ce que** la lentille intraoculaire (5) imprimée exposée au réactif de rupture de chaîne est irradiée, dans le cadre de la désactivation, par un rayonnement électromagnétique (3) d'une longueur d'onde prédéterminée ou d'une plage de longueurs d'onde prédéterminée.

9. Procédé selon l'une quelconque des revendications précédentes, **caractérisé en ce que** le liquide durcissable (2) comprend un agent de réticulation, qui présente deux ou plusieurs groupes (terminaux) acrylate ou méthacrylate pour la polymérisation et entre eux une chaîne moléculaire organique liée de manière covalente, qui présente au moins un groupe photoclivable, qui, lors d'une irradiation par un rayonnement électromagnétique dans la plage de longueurs d'onde UV, est clivé et conduit à un clivage de la chaîne moléculaire, un photoinitiateur étant utilisé dans le liquide durcissable (2), qui peut être activé à une longueur d'onde plus grande que la plage de longueurs d'onde UV, le photoinitiateur étant activé dans le liquide durcissable (2) pendant l'impression tomographique à une longueur d'onde plus grande que la plage de longueurs d'onde UV, de sorte que la chaîne moléculaire n'est pas clivée par l'impression tomographique, la lentille intraoculaire (5) imprimée comportant l'au moins une cavité (6) étant irradiée, dans le cadre de la désactivation, pour cliver le groupe photoclivable par un rayonnement électromagnétique (3) dans la plage de longueurs d'onde UV.

10. Agencement (10) pour la fabrication d'une lentille intraoculaire (5), comprenant :
un récipient transparent (2) au rayonnement électromagnétique (3),
un dispositif de traitement de données (11) qui est conçu pour générer et/ou fournir un ensemble de données (4) à partir d'images d'une lentille intraoculaire (5) présentant au moins une cavité (6), les images contenant des projections de la lentille intraoculaire (5) comportant l'au moins une cavité (6) depuis des directions différentes ;
un dispositif d'impression tomographique (12) qui est équipé pour l'impression tomographique d'un liquide durcissable (2) fourni dans le récipient (1) au moyen d'un rayonnement électromagnétique (3) à partir de l'ensemble de données (4) généré et/ou fourni pour former la lentille intraoculaire (5) comportant l'au moins une cavité (6) ; et **caractérisé en ce que** l'agencement comprend :
au moins un agent de désactivation (15) qui est conçu pour désactiver la capacité de durcissement du liquide durcissable (2) dans l'au moins une cavité (6) ou pour préparer et/ou soutenir la désactivation.
